# EUROPEAN PATENT SPECIFICATION

(11) **EP 3 080 272 B1**
(45) Date of publication and mention of the grant of the patent: **09.08.2017**
(21) Application number: 13826897.4
(22) Date of filing: 13.12.2013
(51) Int. Cl.: C12N 15/115, G01N 33/58

(54) **APTAMER-GATED NANOPARTICLES LATERAL FLOW ASSAYS**
APTAMER GESCHALTETE NANOPATKEL QUERFLUSSASSAY
ANALYSE EN FLUX LATERAL AVEC APTAMER PORTILLONNAGE NANOPARTICULES

(43) Date of publication of application: 19.10.2016
(73) Proprietor: Nanobiz Nanobiyoteknolojik Sistemler Egitim Bilisim Danismanlik Arge San.Tic. Ltd. Sti., 06800 Ankara (TR)
(72) Inventor: OZALP, Veli Cengiz, 06800 Ankara (TR); OKTEM, Huseyin Avni, 06800 Ankara (TR); HERNANDEZ, Frank J., 06800 Ankara (TR); HERNANDEZ, Luiza I., 06800 Ankara (TR); SCHAFER, Thomas, Donostia-San Sebastian (ES)
(74) Representative: Karaahmet, Erdogan
(86) International application number: PCT/TR2013/000373
(87) International publication number: WO 2015/088455

(56) References cited:
- ESTELA CLIMENT ET AL: "Selective, Sensitive, and Rapid Analysis with Lateral-Flow Assays Based on Antibody-Gated Dye-Delivery Systems: The Example of Triacetone Triperoxide", CHEMISTRY - A EUROPEAN JOURNAL, vol. 19, no. 13, 27 February 2013 (2013-02-27), pages 4117-4122, XP055132621, ISSN: 0947-6539, DOI: 10.1002/chem.201300031
- HECHT MANDY ET AL LEVASON BILL ET AL: "Gated hybrid delivery systems:En routeto sensory materials with inherent signal amplification", COORDINATION CHEMISTRY REVIEWS, vol. 257, no. 17, 25 March 2013 (2013-03-25), pages 2589-2606, XP028692868, ISSN: 0010-8545, DOI: 10.1016/J.CCR.2013.03.020
- VELI C. ÖZALP ET AL: "Aptamer-Based Switchable Nanovalves for Stimuli-Responsive Drug Delivery", CHEMISTRY - A EUROPEAN JOURNAL, vol. 17, no. 36, 27 July 2011 (2011-07-27) , pages 9893-9896, XP055132439, ISSN: 0947-6539, DOI: 10.1002/chem.201101403
- CHUN-LING ZHU ET AL: "Bioresponsive Controlled Release Using Mesoporous Silica Nanoparticles Capped with Aptamer-Based Molecular Gate", JOURNAL OF THE AMERICAN CHEMICAL SOCIETY, vol. 133, no. 5, 9 February 2011 (2011-02-09), pages 1278-1281, XP055132442, ISSN: 0002-7863, DOI: 10.1021/ja110094g
- KATIE A EDWARDS ET AL: "Aptamer sandwich assays: human Î+--thrombin detection using liposome enhancement", ANALYTICAL AND BIOANALYTICAL CHEMISTRY, SPRINGER, BERLIN, DE, vol. 398, no. 6, 3 July 2010 (2010-07-03), pages 2645-2654, XP019857516, ISSN: 1618-2650, DOI: 10.1007/S00216-010-3920-4
- XIAOXIAO HE ET AL: "ATP-Responsive Controlled Release System Using Aptamer-Functionalized Mesoporous Silica Nanoparticles", LANGMUIR, vol. 28, no. 35, 4 September 2012 (2012-09-04), pages 12909-12915, XP055106436, ISSN: 0743-7463, DOI: 10.1021/la302767b

## Description

### TECHNICAL FIELD

The invention relates to a method for performing single step assays for the determination of the presence or absence of an analyte in a liquid sample, on a solid surface. The method disclosed, comprise an aptamer coated and signal molecule loaded porous silica particles immobilized on a porous solid material.

Specific interaction of the analyte with the aptamers coated on the silica beads, cause release of the signal molecules which result in a detectable signal on the solid support. Also provided are assay devices for the detection platform.

### BACKGROUND OF THE INVENTION

Lateral flow (LF) immunoassay tests, also known as immunochromatographic strip tests, have been a popular platform for rapid immunoassays since their introduction in the mid-1980s.

The LF format is so versatile that manufacturers of rapid immunoassay tests have developed LF tests for almost any situation where a rapid test is required. In hospitals, clinics, physician offices, and clinical laboratories, LF-based tests are used for the qualitative and quantitative detection of the presence of a specific analyte in a liquid sample.

These test formats generally comprise a lateral flow test strip, such as nitrocellulose or a filter paper, a sample application area, test results area and an analyte specific binding reagent that is bound to some kind of detectable label, such as a colored particle, gold nanoparticle or an enzyme detection system.

The assay consists of several zones, typically constituted by segments made of different materials. When a test is run, sample is added to the proximal end of the strip, the sample pad. The sample migrates through this region to the conjugate pad, where a particulate conjugate has been immobilized. The conjugate comprise of a antibody or aptamer molecule immobilized to a particle. The particle can typically be colloidal gold, or a colored, fluorescent, or paramagnetic monodisperse latex particle.

This particle has been conjugated to one of the specific biological components of the assay, either antigen or antibody depending on the assay format. There are two common formats in LFA: direct and competitive.

The sample re-mobilizes the dried conjugate, and the analyte in the sample interacts with the conjugate as both migrate into the next section of the strip, which is the reaction matrix. This reaction matrix is a porous membrane, onto which the other specific biological component of the assay has been immobilized. These are typically proteins, either a bio-receptor (antibody or aptamer) or antigen, which have been laid down in bands in specific areas of the membrane where they serve to capture the analyte and the conjugate as they migrate by the capture lines. Excess reagents move past the capture lines and are entrapped in the wick or absorbent pad.

Results are interpreted on the reaction matrix as the presence or absence of lines of captured conjugate, read either by eye or using a reader.

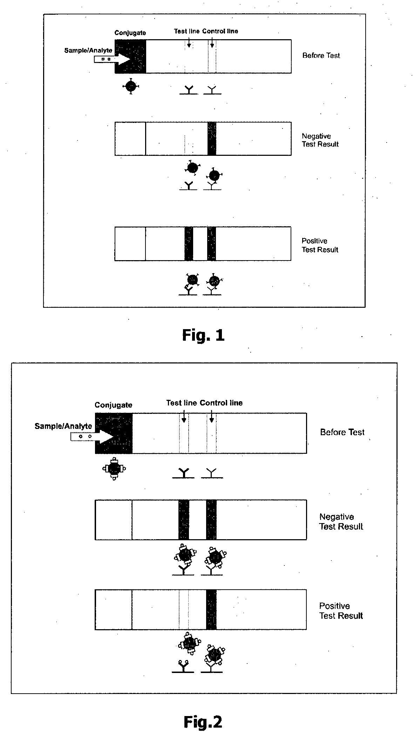

The assay formats can be either direct (sandwich) or competitive (inhibition) and should be able to accommodate qualitative or semi-quantitative determinations. Direct assays are typically used when testing for larger analytes with multiple antigenic sites. Competitive formats are typically used when testing for small molecules with single antigenic determinants, which cannot bind to two antibodies simultaneously. In this format, a positive result is indicated by the absence of a test line on the reaction matrix. A control line should still form, irrespective of the result on the test line. Representative figures for direct or competitive assays are given in Fig. 1 and Fig.2.

A large number of examples of such assay formats are available as literature data and patent applications. Some examples are as follows:
LFA for detection of prostate cancer markers has been defined (Cheng Fanga, Zhencheng Chenb, Lin Lib, Jinhong Xiab. Barcode lateral flow immunochromatographic strip for prostate acid phosphatase determination. Journal of Pharmaceutical and Biomedical Analysis 2011,56, 1035-1040). LFA assays are also defiened for detection of avian influenza virus (Xuepu Li, Donglian Lu, Zonghai Sheng, Kun Chen, Xuebo Guo, Meilin Jin, Heyou Han, A fast and sensitive immunoassay of avian influenza virus based on label-free quantum dot probe and lateral flow test strip. Talanta 2012, 100,1-6). More comphrehensive information can be also found in the following references (Rosen S., Market Trends in Lateral Flow Immunoassays, 35-50, (2009) Lateral Flow Immunoassay, Wong R.C., Harley Y., Tse H.Y., Eds., Humana Press),
Geertruida A. Posthuma-Trumpie, Jakob Korf and Aart van Amerongen. Lateral flow (immuno) assay: its strengths, weaknesses, opportunities and threats. A literature survey Analytical and Bioanalytical Chemistry, 2009, 393:569-582)

Although not as common as antibody based LFA formats, LFA's based on aptamer capture molecules has been also published. It is possible to detect proteins such as thrombin (Hui Xu,Xun Mao,Qingxiang Zeng,Shengfu Wang,Abdel-Nasser Kawde,and Guodong Liu. Aptamer-Functionalized Gold Nanoparticles as Probes in a Dry-Reagent Strip Biosensor for Protein Analysis. Anal. Chem. 2009, 81, 669-675), glycated albumin (Henry John Smith. Aptamer based point-of-care test for glycated albumin, US Patent application No: 20090042237, 2009) and cancer cells (Guodong Liu,Xun Mao,Joseph A. Phillips,Hui Xu,Weihong Tan,and Lingwen Zeng. Aptamer - Nanoparticle Strip Biosensor for Sensitive Detection of Cancer Cells. Anal. Chem. 2009, 81, 10013-10018)

Although LFA is a well-established test platform, it encounters various problems. One major problem is the sensitivity (limit of detection-LOD) for a given sample. Although strategies for signal amplification is possible thus lowering limit of detection is possible, these techniques are not practical and cost effective.

The other problem is the difficulties in developing assays with small molecules. In such assays due to the small nature and limited number of antigenic sites of the analyte it is not possible to establish direct (sandwich) assay formats. In case of competitive assays it is necessary to label or immobilize the small analyte which cause a reduction in the affinity of the assay (Fig 2)

### SUMMARY OF THE INVENTION

The present invention describes a new method of analyte recognition by using membrane immobilized, aptamer coated and signal molecule loaded mesoporous silica beads. The method is applicable to both stationary dot blot assays and lateral flow assay platforms.

It is possible to identify analytes, including but not limited to small molecules (ATP, drugs, hormones, explosives etc.) nucleic acids, carbohydrates proteins, spores, prokaryotic cells (bacterial cells), eukaryotic cells (yeast, tumor cells etc.), and virus particles. The analytes can be detected in a variety of fluid samples including, but not limited to blood, tears, saliva, solid or liquid environmental samples.

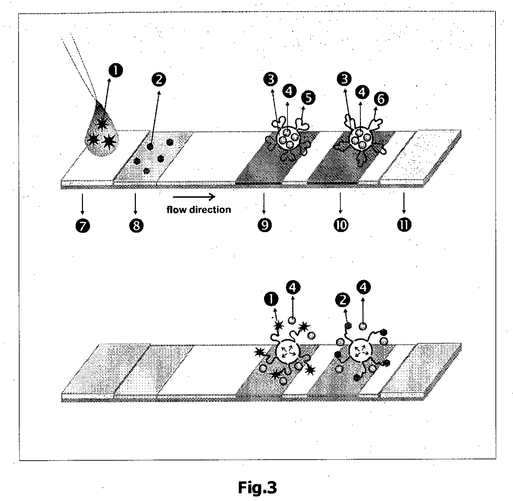

The basic principle of the method is the interaction of the analyte (1) with specific aptamer molecules (2) immobilized on the silica beads (3) loaded with signal molecules (4). The specific interaction between the analyte and the aptamer causes opening of the pores and release of the signal molecules out of the silica beads, which generate a detectable signal (Figure 3). Depending on the type of the signal molecule, the signal can be directly detected (as color, fluorescent etc.) or can be detected after processed by an enzyme (signal like TMB- 3,3',5,5'-tetramethylbenzidine).

### BREIF DESCRIPTION OF THE DRAWINGS

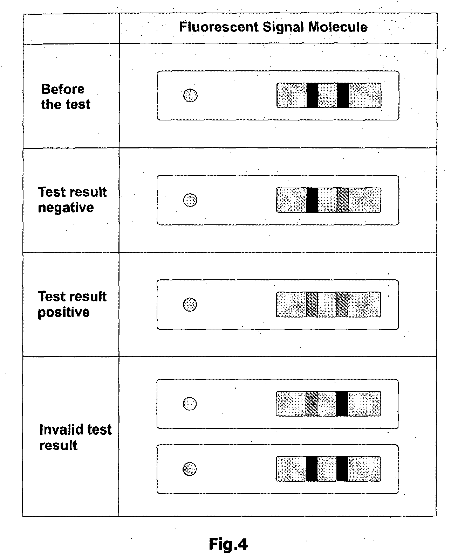

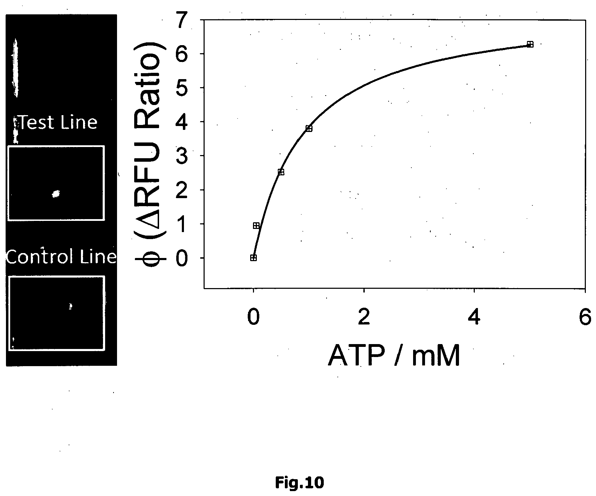

Fig.1 Direct lateral flow assay format. Preferred method for analytes with multiple epitopes.
Fig. 2 Competitive lateral flow assay format. Preferred method for small analytes.
Fig. 3: Lateral flow assay test format utilizing aptamer coated and fluorescence signal molecule loaded silica particles.
   1- Target analyte
   2- Molecule to form a signal at control zone (10)
   3- Silica bead immobilized to the test and control zone (9)
   4- Signal molecule
   5- Specific aptamer for the target analyte (1)
   6- Specific aptamer to 2
   7- Sample application zone
   8- Control molecule zone
   9- Test zone
   10- Control zone
   11- Sample absorption pad
Fig. 4: Top view of the test device and interpretation of the test results when a fluorescence signal molecule is loaded to the silica particles.

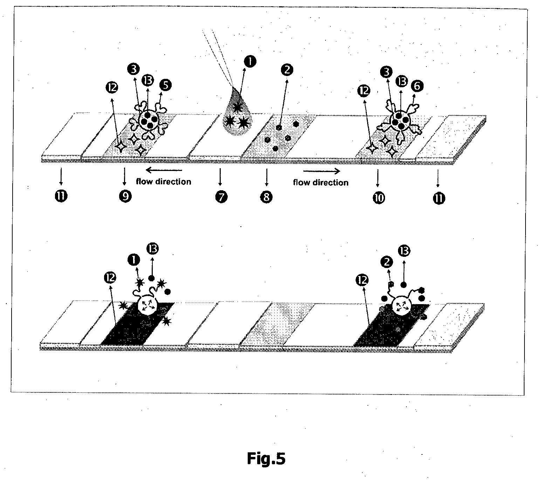

Fig. 5: Lateral flow assay test format utilizing aptamer coated and chromogenic signal molecule loaded silica particles
   1- Target analyte
   2- Molecule to form a signal at control zone (10)
   3- Silica bead immobilized to the test (9) and control zone (10)
   5- Specific aptamer for the target analyte (1)
   6- Specific aptamer to (2)
   7- Sample application zone
   8- Control molecule (2) zone
   9- Test zone
   10- Control zone
   11- Sample absorption pad
   12- Molecule to process the signal molecule (13)
   13-Signal molecule (chromogenic substrate)

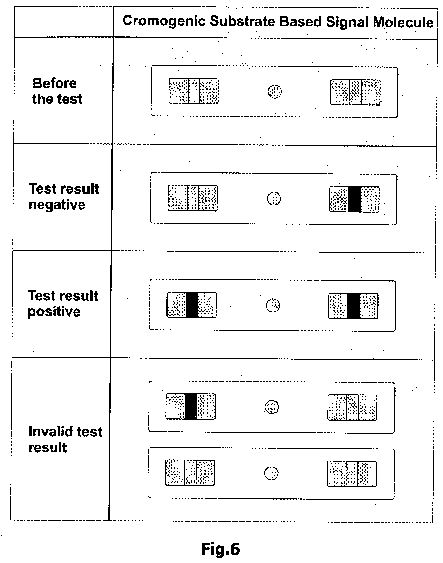

Fig. 6: Top view of the test device And interpretation of the test results when a chromogenic signal molecule is loaded to the silica particles.
Figure-7A: Multiple test format device with 3 channels.
Figure-7B: Top wiev of a test device with 3 channels.
Figure-8A: Multiple test format device with 4 channels
Figure-8B: Top view of multiple test format device with 4 channels
Figure-9A: Multiple test format device with 6 channels
Figure-9B: Top view of multiple test format device with 6 channels
Figure 10: ATP titration results as measured with strips on the left. This is an example of LFA strip showing test line and control line before applaying sample. The picture shows a fluorescent scan by exciting at 480 nm and reading emission at 520 nm (on the left). The results were plotted as ratio of fluorescent values of test line to control line after applying samples with different ATP concentrations. The control line consists of nanoparticles capped with a control aptamer with the same length and the same hairpin shape like ATP binding aptamer, but mutated in four nucleotides (underlined) to obtain a sequence that is not responsive to ATP.

### DETAILED DESCRIPTION OF INVENTION

The invention provides a new assay platform that comprise an aptamer coated and signal molecule loaded silica particle, immobilized on a solid platform, a porous membrane, paper or fabric.

A "signal molecule" refers to a substance or compound that can be detected, particularly by visual, fluorescent or instrumental means. A signal molecule may be, for example, but not limited to, fluorescent molecule such as fluorecein or a pigment produced as a coloring agent or ink, such as Brilliant Blue, 3132 Fast Red 2R and 4230 Malachite Blue Lake.

The "signal molecule" may also be an enzyme substrate, such as, but not limited to, TMB or Bromo-Chloro-Indolylphosphate (BCIP) and Nitro Blue Tetrazolium (NBT/Thiazolyl Blue/Nitro BT). TMB (3,3',5,5'-tetramethylbenzidine) is a chromogenic substrate that yields a blue color when oxidized, typically as a result of oxygen radicals produced by the hydrolysis of hydrogen peroxide by horse radish peroxidase (HRP). For kinetic or non-stopped ELISA assays, the TMB chromogen has maximal absorbances at 370nm and 652nm. TMB is very sensitive ELISA substrate and is more quickly oxidized than other HRP substrates, resulting in faster color development.

The silica bead is a porous nanoparticle that can be chemically synthesized or commercially available form various chemical companies such as Sigma (Sigma, 643645, Silica, mesostructured, MCM-41 type (hexagonal) unit cell size: 4.5-4.8 nm pore size: 0.98 cm cube/ g pore volume, 2.1-2.7 nm pore size).

The aptamer molecule is nucleic acid sequence (DNA or RNA) that exhibits high affinity binding properties and specificity to a given analyte.

The analyte can be any target molecule, cell or virus particle present in a target sample. The analyte can be small molecules such as but not limited to nucleoties (ATP, GTP etc), drugs, pesticides, antibiotics, toxins, explosives, hormones; biomolecules such as short peptides, proteins, carbohydrates, lipids and nucleic acids; bacterial cells, spores, fungal cells; eukaryotic cells such as yeast cells, tumor cells; virus particles and pollen particles.

The sample refers to a fluid such as, but not limited to blood, serum, plasma, saliva, tears, urine, semen, aqueous samples taken from environmental samples such as lakes, rivers, sea, water tanks, municipal, or industrial water sources, runoff water or sewage samples, food samples such as milk, yogurt; hard and soft drinks like wine, fruit juices and vegetable juices. Swap sample from any surface, throat or genital regions can be suspended in liquid solutions and can be also used as a sample.

Sample application zone part is the portion where a liquid sample including the analyte is applied. Depending on the type of the assay format various types of materials (filter paper, glass filter) can be used at this region of the test device to trap undesired molecules where the analyte moves towards the test zone. For instance when a blood sample is applied the blood cells can be hold by this zone. Alternatively molecules like, but not limited to charcoal (to trap phenolic compounds), silica particles (to trap nucleic acids), zeolite particles, aluminum silicate particles; can be included at this region to trap and prevent the interference of undesired molecules at the test zone.

Test zone part is the portion where the analyte interacts with the aptamer coated silica particles and induces the signal formation. Depending on the signal molecule the result can be visualized by naked eye or by the aid of an instrument.

Positive control zone is a test zone to verify the proper functioning of the assay. This zone includes silica particles coated with a particular aptamer, which is activated by a molecule that is by default present at the control molecule zone.

Although not shown in the figures, to estimate the non-specific release of the signal molecule, a negative test zone can be also incorporated to the assay. In such a format, a signal molecule loaded silica particle coated with a non-specific aptamer would be immobilized between test and control zones.

Working principle of the invention is as follows:
The present invention can be used to identify a variety of analytes in a given fluid sample. As shown in Figure 3, the sample (in fluid form) containing the analyte (1) is applied to the sample application zone (7). The sample will move towards the test zone (9) and the sample absorption pad (11) by the capillary action of the membrane support. When the analyte reaches to the test zone (9) it interacts with the specific aptamers (5) coated onto the porous silica beads (3), which are immobilized to the test zone (9). The interaction of the analyte with the aptamer causes the opening of the pores of the silica particles, which in turn causes the release of the signal molecules (4) out of the silica particle thus generating a signal at the test zone.

During the capillary movement of the sample, a control molecule (2) present at the control molecule zone (8) also moves toward the absorption pad (11). When the control molecule reaches to the positive control zone (10) it interacts with its specific aptamer (6) coated on the porous silica particles (3) loaded with the signal molecule (4). This interaction also generates a signal, which indicates the proper functioning of the assay. Alternatives for control molecule includes but not limited to a well defined analyte-aptamer pairs like:
ATP - CACCTGGGGGAGTATTGCGGAGGAAGGTT (Huizenga, Biochemistry. 1995 17;34(2):656-65) Argininamide- CGACCAACGTGTCGCCTGGTCG (Harada K, Frankel AD (1995) EMBO J 14: 5798-5811)
Thrombin- GGTTGGTGTGGTTGG (Bock LC, Griffin LC, Latham JA, Vermaas EH, Toole JJ. Nature 1992;355:564-566.)

Alternatively, complementary nucleic acid sequences where one of the complementary sequence is coated on the silica bead and the other single strand complementary sequence is applied to the control molecule zone (8).

Depending on the nature of the signal molecule, type of the signal differs. In case of a florescent signal molecule, presence of analyte in the test sample causes a reduction in the fluorescent intensity due to the release of entrapped signal molecules at the test zone. Figure 4 shows different possibilities and interpretation of the assay formats when a fluorescent signal is loaded into the silica beads.

The test formats using fluorescence signal molecules, needs instrumentation to be evaluated and it is not possible to evaluate the test results by visual inspection.

The present invention can be also used to obtain test results, which can be evaluated without the use of any instrument. In such test formats (Figure 5) the results can be evaluated by visual inspection by naked eyes.

The assay format comprise a single strip where the sample to be evaluated is applied to the sample application pad (8) which is located at the center of the strip. The sample with the analyte (1) moves towards sample absorption pads (11) present at both ends of the strip by the capillary action. When the analyte reaches to the test zone (10) it interacts with the specific aptamers (5) coated onto the porous silica beads (3) immobilized at the test zone (9) The interaction of the analyte with the specific aptamer (5) causes the opening of the pores of the silica particles, which in turn causes the release of the signal molecule (13). The signal molecule (13) in this case can be a chromogenic molecule or a chromogenic substrate for an enzyme (12), which is also immobilized at the test zone (10) or close vicinity of the test zone.

In case of a chromogenic signal molecule a visual signal directly appears at the test zone after the release of the chromogenic molecule from the silica beads. This signal can be detected by visual inspection of the test zone (Figure 6)

If the signal molecule is a chromogenic substrate, after the release from the silica beads, the substrate is processed by the enzyme (12) present at the test zone to form a visual signal that can be detected by visual inspection of the test zone (Figure 5).

In order to evaluate the proper functioning of the test, a positive control zone (10) is also established at the opposite side of the strips. During the capillary movement of the sample towards the absorption pad (12), a control molecule (2) present at the control molecule zone (8) also moves toward the absorption pad (12). When the control molecule reaches to the positive control zone (10) it interacts with its specific aptamer (6) coated on the porous silica particles (3) loaded with the signal molecule (13). This interaction also causes the release of the signal molecule out of the porous silica particles (3) at the positive control zone (10) which, in turn forms a direct visual signal (in case the signal molecule is a chromogenic molecule) or after processes by the enzyme (12) located at the positive control zone (in case the signal molecule is a chromogenic substrate). This signal is an indicator of the proper functioning of the assay. Possible assay outcomes and their interpretation is shown in Figure 6.

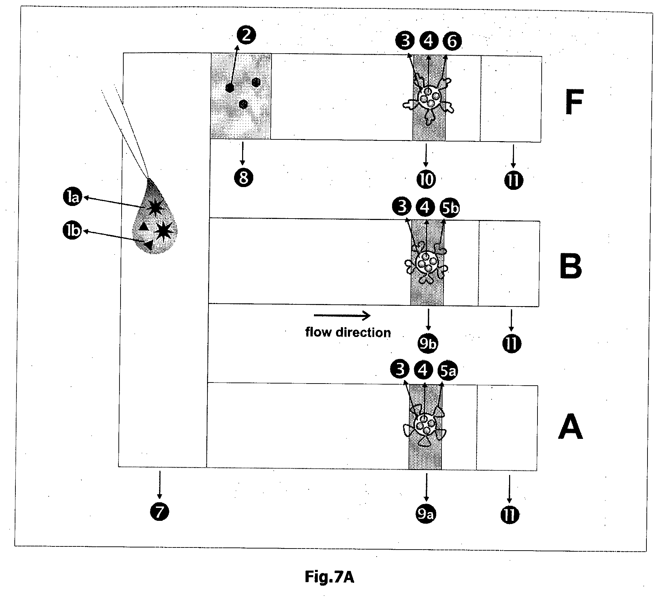

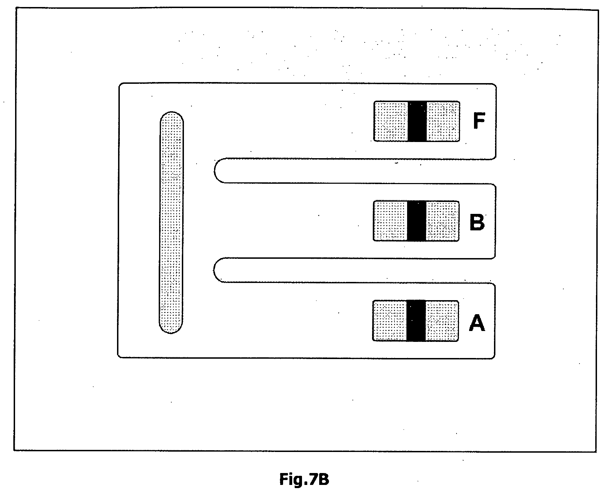

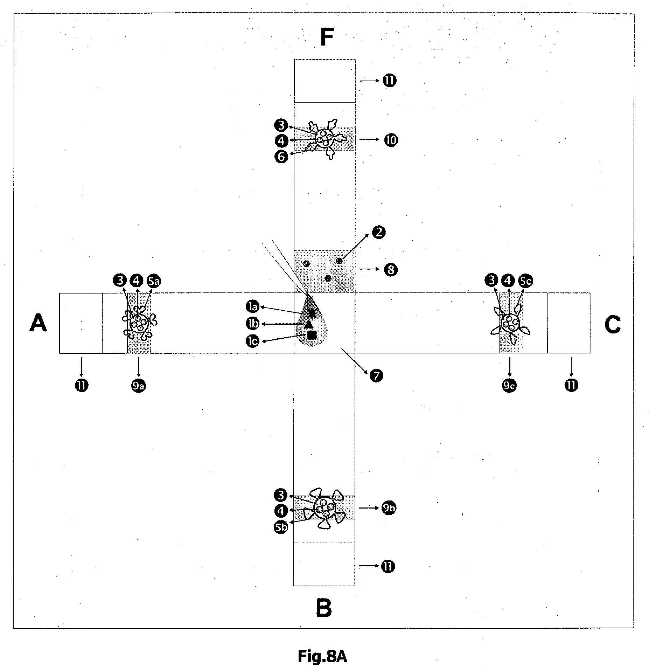

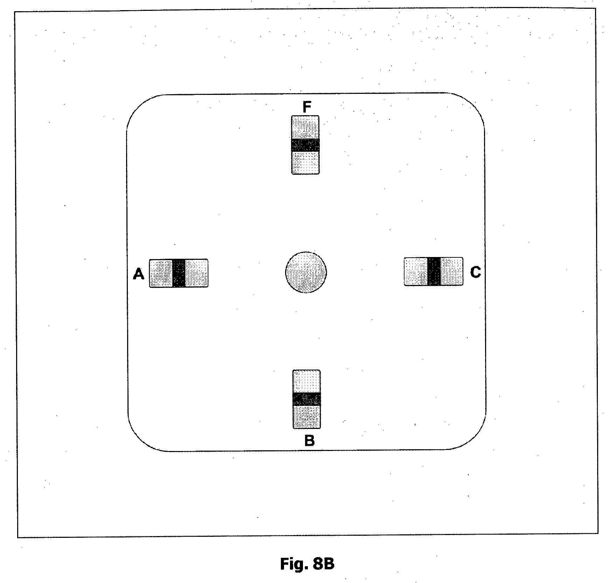

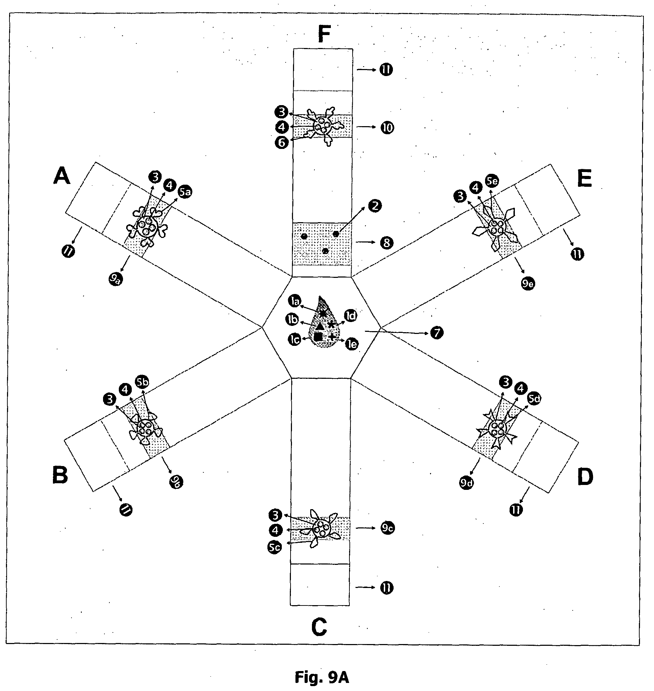

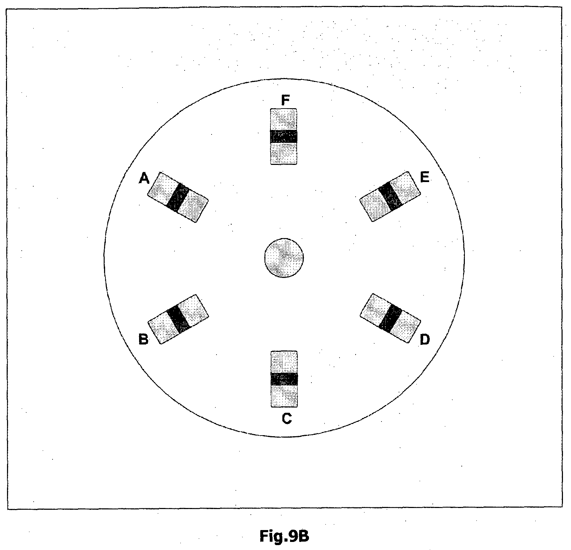

Present invention can be also used to detect various analytes at the same time (Figure 7-9). In such test formats there is a single sample application zone (7), which is connected to multiple test channels (A-E) and a control channel (F). Each test channel contains a test zone (9a-9e). Depending on the number of different analytes to be detected the number of the test channels can be multiplied. Each test zone (9a-e) contains silica beads coated with different specific aptamers for a given analyte that are intended to be analyzed by the test device. Multiple test zone test formats also contain a control channel (F). At the entrance of the test channels there is the control molecule pad (8) with the control molecule (2). The control channel is also equipped with signal molecule loaded silica beads coated with aptamers specific to the control molecule. These beads are immobilized to the control zone (10). All of the channels have a sample adsorption pad at their distal ends (11).

Present invention has versatile application areas including but not limited to health, food security, homeland security, bioterrorism, environmental monitoring, veterinary applications, beverage industry and others. Although it is not limited with those, some representative examples for the application of the present invention is given below.

### SMALL MOLECULE DETECTION (ATP)

The present invention may be used to identify ATP molecules in a given sample. The silica particles with signal molecule inside and aptamers on the surface covering the pore openings were previously reported to detect a small molecule in solution form (Ozalp, V.C. & Schafer, T. Chem. Eur. J. 2011, 17, 9893 - 9896; Chun-Ling Zhu; Chun-Hua Lu; Xue-Yuan Song; Huang-Hao Yang; Xiao-Ru Wang; J. Am. Chem. Soc. 2011, 133, 1278-1281). Fluorescein molecules (signal molecule) was loaded in silica nanoparticle porous containers and covered with ATP responsive aptamer sequences as described in Ozalp and Schafer (Ozalp, V.C. & Schafer, T. Chem. Eur. J. 2011, 17, 9893 - 9896). ATP binding aptamer sequence was originally selected by Huizenga (D. E. Huizenga, J. W. Szostak, Biochemistry 1995, 34, 656 -665). The aptamer sequence was converted into a molecular-beacon-type hair pin structure by adding additional nucleotides at one end of the sequence. The final hairpin structure was as follows:
**(5'-CACCTGGGGGAGTATTGCGGAGGAAGGTT*CCAGGTG*-NH2-3')** (italic sequence is the added nucleotides for converting the aptamer to hairpin)

Mesostructured silica particles (MCM-41) of hexagonal architecture with a unit size of 4.6 to 4.8 nm and an average pore size of 2.3 to 2.7 nm were employed for preparing ATP resposive release system. The aptamer hairpin structures were covalently conjugated to the silica surface of nanoparticles. The duplex neck region of the hairpin structure of the ATP-binding aptamer DNA when attached to the surface of nanoparticles, could successfully block the release of guest molecules in mesoporous silica particles. In the present invention, fluorecein loaded silica nanoparticles capped with ATP recognizing aptamer sequences in hairpin structure were immobilized on test regions of LFA strips through a linker (Ozalp V.C., Analyst, 2013, 138, 4255) or by applying polyacrylamide embedding. Figure 10 shows that when samples containing ATP were applied on the sample pad, ATP molecules flow through test and control lines. Since ATP causes opening of aptamer gates, fluorescein inside the nanopores are released and washed by flowing buffer. The release of fluorescein can be monitored by quantifying the decrease of fluorescein signal in the test line as described in detail elsewhere (Ozalp V.C., Analyst, 2013, 138, 4255). The absolute decrease of fluorescein was plotted against concnetration of ATP samples to obtain a binding curve fitted to Langmuire type binding kinetics (Fig. 11). **5'-CACCTAGGAGAGTAATGCCGAGGAAGGTT*CCAGGTG-*NH2-3'**
(Underlined are mutated nucleotides)

### VIRUS PARTICLE DETECTION (HIV)

As an example to the application in the health sector, the invention can be used to detect HIV viruses in blood samples and body fluids. For such applications few drops of the sample (blood or saliva) is applied to the sample pad. The sample pad contains specific materials, which absorbs or filtrates the blood cells. To achieve the specific detection, the signal molecule loaded porous silica beads scan be coated with HIV specific aptamers. For example, RNA aptamer recognizing TAT protein as shown below:
TAT aptamer: **ACGAAGCUUGAUCCCGUUUGCCGGUCGAUCGCUUCGA**

Similarly, DNA aptamer sequences were selected to bind specifically to HIV reverse transcriptase enzyme.

5'-
AGCAGCACAGAGGTCAGATGGCAGGTTTCGACGTACAATGCTATGGAGGCTTTATGATCGCC
TATGCGTGCTACCGTGAA-3'

An example application to veterinary field is given below.

Bovine viral diarrhea is a viral disease of cattle and other ruminants which reduces productivity and increases death loss. It is caused by the bovine viral diarrhea virus (BVDV). BVDV is a member of the pestivirus genus. There are four recognized species within the pestivirus genus. These species are BVDV-1, BVDV-2, border disease virus of sheep and classical swine fever virus, previously known as hog cholera virus.

In the literature aptamers specific to bovine viral diarrhea virus (BVDV) type 1 virus has been defied (Jee-Woong Park, Su Jin Lee, Eun-Jin Choi, Jaejo Kim, Jae-Young Song, Man Bock Gu Biosensors and Bioelectronics 51 (2014) 324-329). Two of such aptamer sequences are given below.

5'-Bio-
CGTACGGAATTCGCTAGCTGCGCATCCACAAATGTATTGTCGGGGGATGGATCCGAGCTCCA
CGTG-3'

5'-Thiol-
CGTACGGAATTCGCTAGCTGCGCATCCACAAATGTATTGTCGGGGGATGGATCCGAGCTCCA
CGTG-3'

The invention can be used fort the detection of BVDV type 1 on a lateral flow assays. As described in different parts of the document the mesostructured silica particels can be loaded with a signal molecule like 3,3',5,5'-Tetramethylbenzidine (TMB) and lateral flow strips can be prepared as shown in Figure 3.

These silica particles with signal molecule inside and aptamers on the surface covering the pore openings will be applied on the lateral flow strips as shown in Figures 1 and 3. If the silica particles are loaded by TMB, the test line will contain HRP. Sample will be prepared with proper amount of H₂O₂ and applied to the sample pad. If the sample contains the virus particles, then the aptamer on silica particle surface will specifically interact with the aptamer and left the pores open. As a result there will be a release of TMB, and the blue color forming oxidation reaction will occur with the enzyme HRP and H₂O₂ inside the sample.

### EUKARYOTIC CELL DETECTION (TUMOR CELLS)

Another application is identification of a target cell like a tumor cell. For such an application signal molecule loaded silica nanoparticles would be coated by an aptamer specific to nucleolin protein. The sequence of the nucleolin aptamer AS1411 was previously published (P. J. Bates, D. A. Laber, D. M. Miller, S. D. Thomas and J. O. Trent, Exp. Mol. Pathol., 2009, 86, 151-164). The AS1411 aptamer is a G-quadruplex forming a 26-base DNA sequence that has high affinity and specificity for nucleolin protein. The aptamer sequence is as follows:
**5'-GGTGGTGGTGGTTGTGGTGGTGGTGG-3'**

Nucleolin is a multifunctional protein that can interact with both DNA and RNA molecules. It is present in the nucleolus and cytoplasm of most healthy cells and its expression is highly elevated on the surface of rapidly proliferating cancer cells. Therefore, in the case of the presence of a tumor cell in an particular sample, the cells interacts with the aptamer coated silica particles which causes the release of the signal molecule. This in turn causes the generation of a detectable signal on the lateral flow device.

### BACTERIAL PATHOGEN DETECTION (SALMONELLA DETECTION)

An examlpe for food security area is as follows. Salmonellae are a significant cause of food borne illness world-wide. Among a multitude of epidemic enteric bacterial infections, salmonellosis is one of the most frequently reported bacterial foodborne diseases and is a major economic and public health concern worldwide. Of particular concern is salmonellosis caused by multidrug-resistant strains such as *Salmonella enterica* serovar Typhimurium or *S*. *enterica* serovar Newport (Gupta, A.; Fontana, J.; Crowe, C.; Bolstorff, B.; Stout, A.; Van Duyne, S.; Hoekstra, M. P.; Whichard, J. M.; Barrett, T. J.; Angulo, F. J.: J. Infect. Dis. 2003, 188, 1707 - 1716). In addition, *S*. *typhimurium* is a major causative agent of gastroenteritis (characterized by diarrhea, cramps, vomiting, and often fever) in many areas (Jones, B. D.; Ghori, N.; Falkow, S.: J. Exp. Med. 1994, 180, 15-23). Salmonellae species are also important bacterial pathogens in food animal species including chickens, cows, turkeys and pigs. Amongst non-human cases, *S*, *typhimurium* is reported as the most causative agent of infections. The present invention can be also used to detect *S.typimirum* presence in a given sample.

Silica particles were incubated overnight in phosphate buffered saline solution loaded with the signal molecule (TMB or FAM). Silanization of the surface will be followed by immobilization of the specific aptamer molecules as reported in the literature (Raghavendra Joshi, Harish Janagama, Hari P. Dwivedi, T.M.A. Senthil Kumar, Lee-Ann Jaykus,Jeremy Schefers, Srinand Sreevatsan. Molecular and Cellular Probes 23 (2009) 20-28.7). On of such aptamer molecule sequence is as follows:
**5'-TAT GGC GGC GTC ACC CGA CGG GGA CTT-3'**

These silica particles with signal molecule inside and aptamers on the surface covering the pore openings will be applied on the lateral flow strips as shown in Figures 1 and 3. If the silica particles are loaded by TMB, the test line will contain HRP. Sample will be prepared with proper amount of H₂O₂ and applied to the sample pad. If the sample contains the Salmonella species, then the aptamer on silica particle surface will specifically interact with the aptamer and left the pores open. As a result there will be a leakage of TMB, and the blue color forming oxidation reaction will occur with the HRP in close position and H₂O₂ inside the sample. On the other hand, in the absence of Salmonella in the sample solution, no pore opening will happen thus no signal formation occurs.

### PROTEIN DETECTION (RICIN)

The invention can be used to identify presence of target protein molecules in sample of interest. One such application would be detection of a bioterror agent ricin in a given sample. If the provided sample is not fluid, it might be suspended in phosphate buffered saline (PBS) solution and then applied to the test device. All of the steps of the procedures would be the same as explained previously in different parts of this document, except for the aptamers that is coated on the surface of the silica beads. The aptamer molecule would be a sequence that is already published in the literature (Elise A. Lamont, Lili He, Keith Warriner, Theodore P. Labuzab and Srinand Sreevatsan, Analyst, 2011,136, 3884-3895). The sequence of the aptamer for ricin is as follows:
**5' ACACCCACCGCAGGCAGACGCAACGCCTCGGAGACTAGCC 3'**

## Claims

1. Lateral Flow assay platform comprising application zone (7), at least one test zone (9) where aptamer (5) coated and signal molecule (4) loaded particle (3) is immobilized, control zone (8) where a control molecule (2) is immobilized and positive control zone (10) wherein an analyte interacts with the aptamer (5) that causes the opening of the pores of the silica particles which in turn causes the release of the signal molecule (4)

2. Lateral Flow assay platform according to claim 1 where signal molecule (4) is fluorescent molecule.

3. Lateral Flow assay platform according to claim 2 where signal molecule (4) is fluorescein

4. Lateral Flow assay platform according to claim 1 where signal molecule (4) is chromogenic molecule

5. Lateral Flow assay platform according to claim 4 where signal molecule (4) is enzyme substrate

6. Lateral Flow assay platform according to claim 5 where signal molecule (4) is TMB (3,3',5,5'-tetramethylbenzidine)

7. Lateral Flow assay platform according to claim 4 where signal molecule (4) is Bromo-Chloro-Indolylphosphate (BCIP) or Nitro Blue Tetrazolium (NBT/Thiazolyl Blue/Nitro BT)

8. Lateral Flow assay platform according to claim 1 where particle (3) is porous silica bead.

9. Lateral Flow assay platform according to claim 1 where control molecule (2) is ATP, Argininamide, thrombin or a single stranded nucleic acid sequence complementary to nucleic acid sequence coted on the silica beads (3) present at the control zone (10)

10. A method for detecting an analyte by using Lateral Flow assay platform according to claim 1 comprises,
a) Applying sample to application zone (7)
b) Interaction of analyte (1) with specific aptamer (5) coated silica beads (3) on test zone (9)
c) Opening the pores of silica particles (3)
d) Release of the signal molecule (4)
e) Generating a signal at the test zone (9)
f) forwarding control molecule (2) on control zone (8) to positive control zone (10)
g) Interaction of control molecule (2) with specific aptamer (6) coated silica beads (3) on positive control zone (10)

11. A method according to claim 10 where sample is blood, serum, plasma, saliva, tears, urine, semen.

12. A method according to claim 10 where sample is aqueous samples taken from lakes, rivers, sea, water tanks, municipal, or industrial water sources, runoff water or sewage samples,

13. A method according to claim 10 where sample is food samples from meat, vegetables, fruits, milk, yogurt; hard and soft drinks, fruit juices and vegetable juices.

14. A method according to claim 10 where sample from throat or genital regions.

15. A method according to claim 10 where analyte is selected from small molecule, nucleotides (ATP, GTP etc), drugs, narcotic drugs, pesticides, antibiotics, toxins, explosives, hormones, biomolecules, short peptides, proteins, carbohydrates, lipids and nucleic acids, bacterial cells, spores fungal cells eukaryotic cells, tumor cells. virus particles or pollen particles.

## Patentansprüche

1. Seitliche Flussanalyseplattform bestehend aus einer Applikationszone (7), mindestens einem Testgebiet (9), wo Aptamer (5) angestrichen und Signalmolekül (4) eingeladene Teilchen (3) unbeweglich gemacht wird, einer Kontrollzone (8), wo ein Kontrollmolekül (2) unbeweglich gemacht wird und einer positiven Kontrollzone (10), worin ein Analyt mit Aptamer (5) einwirkt, das verursacht, daß die Poren von Siliziumteilchen eröffnet werden, wodurch wiederum Signalmolekül (4) freigegeben wird.

2. Seitliche Flussanalyseplattform nach Anspruch 1, wo Signalmolekül (4) Fluoreszenzmolekül ist.

3. Seitliche Flussanalyseplattform nach Anspruch 2, wo Signalmolekül (4) Fluoreszein ist

4. Seitliche Flussanalyseplattform nach Anspruch 1, wo Signalmolekül (4) Farbenmolekül ist

5. Seitliche Reihenanalyseplattform nach Anspruch 4, wo Signalmolekül (4) Enzym-substrat ist

6. Seitliche Flussanalyseplattform nach Anspruch 5, wo Signalmolekül (4) TMB (3,3',5,5'-Tetramethylbenzidin) ist

7. Seitliche Flussanalyseplattform nach Anspruch 4, wo Signalmolekül (4) Brom-Chlor-Indolphosphat (BCIP) oder Nitro Blau Tetrazolium (NBT/Thiazolyl Blau/Nitro BT) ist

8. Seitliche Reihenanalyseplattform nach Anspruch 1, wo Teilchen (3) poröse Siliziumperle ist.

9. Seitliche Reihenanalyseplattform nach Anspruch 1, wo Kontrollmolekül (2) ATP ist, Argininamide, Thrombin oder eine einzig gestrandete Nukleinsäurenfolge ergänzend zur Nukleinsäurenfolge vorbeigegangen auf der Siliziumperle (3) sind in Kontrollzone (10) vorhanden

10. Eine Methode für Entdeckung eines Analyten durch Verwendung von seitlicher Reihenanalyseplattform nach Anspruch 1 besteht aus,
a) Auflegung der Probe zur Applikationszone (7)
b) Einwirkung des Analyten (1) mit spezifischer Aptamer (5) angestrichene Siliziumperlen (3) auf Testgebiet (9)
c) Eröffnung der Poren von Siliziumteilchen (3)
d) Freigabe vom Signalmolekül (4)
e) Erzeugung eines Signals an Testgebiet (9)
f) Nachsendung des Kontrollmoleküls (2) auf Kontrollzone (8) zur positiven Kontrollgebiet (10)
g) Einwirkung des Kontrollmoleküls (2) mit spezifischer Aptamer (6) angestrichene Siliziumperlen (3) auf positiver Kontrollzone (10)

11. Eine Methode nach Anspruch 10, wo Probe Blut, Serum, Plasma, Speichel, Träne, Urin, Samen ist.

12. Eine Methode nach Anspruch 10, wo Probe wässrige Proben ist, die von Seen, Flüssen, Meer, Wassertanken, städtische oder industrielle Wasserquellen, Ablaufwasser oder Abwasser aufgenommen sind,

13. Eine Methode nach Anspruch 10, wo Probe Nährstoffe vom Fleisch, Gemüsen, Obste, Milch, Joghurt; scharfe und alkoholfreie Getränke, Frucht- und Gemüsesäfte ist.

14. Eine Methode nach Anspruch 10, wo Probe vom Hals oder geschlechtlichen Gebiete ist.

15. Eine Methode nach Anspruch 10, wo Analyt vom Kleinmolekül, Nukleotiden (ATP, GTP usw.), Rauschgifte, Narkotikum, Schädlingsbekämpfungsmitteln, Antibiotika, Giftstoffe, Explosivstoffe, Hormone, Biomoleküle, Kurzpeptide, Proteine, Kohlenhydrate, Lipide und Nukleinsäuren, Bakterienzellen, Sporen, Pilzzellen, Eukaryonten, Tumorzellen, Viruspartikeln oder Pollenpartikeln ausgewählt ist.

## Revendications

1. La plate-forme d'essai de flux latéral comprenant la zone d'application (7), au moins une zone d'essai (9) où particule (3) revêtue par l'aptamère (5) et chargée de la molécule de signal (4) est immobilisée, zone de contrôle (8) où une molécule de contrôle (2) est immobilisée et une zone de contrôle positive (10) dans laquelle un analyte interagit avec l'aptamère (5) qui provoque l'ouverture des pores des particules de silice qui provoque à son tour la libération de la molécule de signal (4).

2. Plate-forme d'essai de flux latéral selon la revendication 1, dans laquelle la molécule de signal (4) est une molécule fluorescente.

3. Plate-forme d'essai de flux latéral selon la revendication 2, dans laquelle la molécule de signal (4) est la fluorescéine

4. Plate-forme d'essai de flux latéral selon la revendication 1, dans laquelle la molécule de signal (4) est une molécule chromogène

5. Plate-forme d'essai de rangée latérale selon la revendication 4, dans laquelle la molécule de signal (4) est un substrat enzymatique

6. Plate-forme d'essai de flux latéral selon la revendication 5, dans laquelle la molécule de signal (4) est TMB (3,3', 5,5'-tétraméthylbenzidine)

7. Plate-forme d'essai de flux latéral selon la revendication 4, dans laquelle la molécule de signal (4) est le bromo-chloro-lindolylphosphate (BCIP) ou le tétrazolium nitrique bleu (NBT / Thiazolyl Blue / Nitro BT)

8. Plate-forme d'essai de rangée latérale selon la revendication 1, dans laquelle la particule (3) est une perle de silice poreuse.

9. Plate-forme d'essai de rangée latérale selon la revendication 1, dans laquelle la molécule de contrôle (2) est l'ATP, l'argininamide, la thrombine ou une séquence d'acide nucléique à un seul brin complémentaire de la séquence d'acide nucléique cité sur les perles de silice (3) présentes dans la zone de contrôle (10)

10. Un procédé pour détecter un analyte en utilisant une plate-forme d'essai de rangée latérale selon la revendication 1, comprenant,
a) Application de l'échantillon à la zone d'application (7)
b) Interaction de l'analyte (1) avec des perles de silice (3) revêtues d' un aptamère spécifique (5) sur la zone d'essai (9)
c) Ouverture des pores de particules de silice (3)
d) Libération de la molécule de signal (4)
e) Génération d'un signal à la zone d'essai (9)
f) Transférer la molécule de contrôle (2) sur la zone de contrôle (8) vers une zone de contrôle positif (10)
g) Interaction de la molécule de contrôle (2) avec des perles de silice (3) revêtues d'un aptamère spécifique (6) sur la zone de contrôle positive (10)

11. Procédé selon la revendication 10, dans lequel l'échantillon est le sang, le sérum, le plasma, la salive, les larmes, les urines, le sperme.

12. Procédé selon la revendication 10, dans lequel l'échantillon est constitué d'échantillons aqueux prélevés dans les lacs, les rivières, la mer, les réservoirs d'eau, les sources d'eau municipales ou industrielles, les eaux de ruissellement ou les échantillons d'eaux usées,

13. Procédé selon la revendication 10, dans lequel l'échantillon est un échantillon de nourriture provenant de viande, de légumes, de fruits, de lait, de yogourt; des boissons alcoolisées et les boissons gazeuses, les jus de fruits et les jus de légumes.

14. Procédé selon la revendication 10, où un échantillon est de la gorge ou des régions génitales.

15. Procédé selon la revendication 10, dans lequel l'analyte est choisi parmi la petite molécule, les nucléotides (ATP, GTP, etc.), les médicaments, les stupéfiants, les pesticides, les antibiotiques, les toxines, les explosifs, les hormones, les biomolécules, les peptides courts, les protéines, les hydrates de carbone, les lipides et les acides nucléiques , Cellules bactériennes, les cellules des spores fongiques, les cellules eucaryotes, cellules tumorales, les particules virales ou particules de pollen.
